# EUROPÄISCHE PATENTSCHRIFT

(11) **EP 3 748 652 B1**
(45) Veröffentlichungstag und Bekanntmachung des Hinweises auf die Patenterteilung: **10.07.2024**
(21) Anmeldenummer: 20177075.7
(22) Anmeldetag: 28.05.2020
(51) Int. Cl.: G21F 1/10, A61B 6/10, B65B 29/10, G21F 1/12, G21F 3/00, B65B 11/50, B65B 51/22, B65B 31/00, A61B 6/04

(54) **VERFAHREN ZUM HERSTELLEN EINES STRAHLENSCHUTZELEMENTS, STRAHLENSCHUTZELEMENT UND STRAHLENSCHUTZEINRICHTUNG**
METHOD OF MANUFACTURING A RADIATION PROTECTION ELEMENT, RADIATION PROTECTION ELEMENT AND RADIATION PROTECTION DEVICE
PROCÉDÉ DE FABRICATION D'UN ÉLÉMENT DE RADIOPROTECTION, ÉLÉMENT DE RADIOPROTECTION ET DISPOSITIF DE RADIOPROTECTION

(30) Priorität: 04.06.2019 DE 102019003954
(43) Veröffentlichungstag der Anmeldung: 09.12.2020
(73) Patentinhaber: MAVIG GmbH, 81829 München (DE)
(72) Erfinder: KÖSTLMEIER, Manfred, 85640 Putzbrunn (DE); KLEIN, Frank, 85435 Erding (DE); STANGL, Felix, 81825 München (DE)
(74) Vertreter: Vossius & Partner Patentanwälte Rechtsanwälte mbB

(56) Entgegenhaltungen:
- WO-A1-2016/174111
- WO-A1-98/53461
- CN-A- 1 852 944

## Beschreibung

### GEBIET DER ERFINDUNG

Die Erfindung betrifft Strahlenschutzelemente und Verfahren zu deren Herstellung. Die Erfindung betrifft insbesondere Strahlenschutzelemente, die eine Hülle aus einem kunststoffhaltigen Material und ein in der Hülle angeordnetes Strahlenschutzmaterial aufweisen.

### HINTERGRUND

Strahlenschutzeinrichtungen zur Verwendung beispielsweise in der interventionellen Radiologie dienen bei Diagnostik und Therapie sowie in Operationsräumen zum Schutz von beteiligtem Personal gegen auftretende Strahlung, insbesondere Röntgenstrahlung. Derartige Strahlenschutzeinrichtungen können beispielsweise einen Untertisch-Strahlenschutz mit an einem Patiententisch montierbaren Lamellen oder andere an einem Patiententisch montierbare Strahlenschutzelemente aufweisen. Es sind auch andere Ausgestaltungen von Strahlenschutzeinrichtungen bekannt, beispielsweise in Form eines verfahrbaren Strahlenschutzwagens, der ein Unterteil mit flexiblen Lamellen und ein Oberteil mit abgewinkelten Lamellen aufweisen kann.

Die DE 10 2009 025 380 A1 offenbart eine Strahlenschutzanordnung, die mindestens eine Lamelle aus einem Strahlenschutzmaterial und eine zugehörige Befestigungsvorrichtung aufweist.

Die DE 10 2015 208 829 A1 offenbart eine Strahlenschutzanordnung zur Anbringung an einer Trägerschiene, die an einer Seite eines Behandlungstisches angebracht ist.

Die WO 98/53461 A1 offenbart Produkte, die Schwermetalle enthalten und die als Strahlenschutz verwendbar sind.

Die WO 2016/174111 A1 offenbart eine Produktverpackung mit einer Verschlusszone, die aufbrechbar ist, um ein Produkt mit einem Mittel in Kontakt zu bringen.

Aus Hygienegründen, zum Schutz des Strahlenschutzmaterials und aus weiteren Gründen kann es wünschenswert sein, dass Strahlenschutzelemente eine Hülle und ein im Innenvolumen der Hülle angeordnetes Strahlenschutzmaterial aufweisen. Die Hülle kann eine Kunststoffhülle sein.

Häufig besteht das Problem, dass eine Außenfläche der Hülle eine unerwünschte Faltenbildung aufweist. Derartige Falten können die Montage des Strahlenschutzelements erschweren. Oxidationsprozesse des Strahlenschutzelements können durch Lufteinschlüsse in den Falten gefördert werden. Eine Erkennung, ob das Strahlenschutzmaterial korrekt in der Hülle positioniert ist, kann durch derartige Falten ebenfalls erschwert werden.

### ZUSAMMENFASSUNG DER ERFINDUNG

Es ist eine Aufgabe der Erfindung, verbesserte Strahlenschutzelemente mit einer Hülle und wenigstens einem in der Hülle angeordneten Strahlenschutzmaterial sowie verbesserte Herstellungsverfahren für derartige Strahlenschutzelemente anzugeben. Es ist insbesondere eine Aufgabe der Erfindung, derartige Strahlenschutzelemente und Herstellungsverfahren bereitzustellen, die das Risiko verringern, dass durch eine Faltenbildung in der Hülle die Montage der Strahlenschutzelements an einem Patiententisch oder einer anderen Struktur erschwert wird, Oxidationsprozesse in der Hülle gefördert werden und/oder die Prüfung erschwert wird, ob das Strahlenschutzmaterial korrekt in der Hülle positioniert ist.

Erfindungsgemäß werden ein Verfahren, Strahlenschutzelemente und eine Strahlenschutzeinrichtung mit den in den unabhängigen Ansprüchen angegebenen Merkmalen bereitgestellt. Die abhängigen Ansprüche definieren Ausführungsbeispiele.

Ein erfindungsgemäßes Verfahren zum Herstellen eines Strahlenschutzelements weist den Schritt eines Anordnens wenigstens eines Strahlenschutzmaterials zwischen wenigstens zwei Lagen wenigstens eines kunststoffhaltigen Elements auf. Wenigstens ein Teil von Gas, das zwischen den wenigstens zwei Lagen vorhandenen ist, kann entfernt werden. Dies kann beispielsweise durch Anpressen einer oder beider der Lagen oder durch Erzeugen eines Teilvakuums geschehen. Alternativ oder zusätzlich können die wenigstens zwei Lagen mit dem dazwischen angeordneten wenigstens einen Strahlenschutzmaterial in eine Schutzgasatmosphäre eingebracht werden. Die wenigstens zwei Lagen werden dann miteinander verbunden.

Das wenigstens eine Strahlenschutzmaterial kann eine Lage oder mehrere Lagen eines Strahlenschutzmaterials, beispielsweise eine Lage oder mehrere Lagen eines flächigen Strahlenschutzmaterials, aufweisen.

Bei dem Verfahren kann in einem Bereich zwischen den wenigstens zwei Lagen ein Gasdruck von einem Ausgangsdruck auf einen Bearbeitungsgasdruck reduziert werden, während die wenigstens zwei Lagen miteinander verbunden werden.

Der Bearbeitungsgasdruck wird so gewählt, dass im Inneren einer durch Verbinden der wenigstens zwei Lagen gebildeten Hülle des Strahlenschutzmaterials ein Gasdruck von weniger als 1 bar bei einer Temperatur von 23°C herrscht, oder die wenigstens zwei Lagen werden in einer Schutzgasatmosphäre miteinander verbunden. Die Schutzgasatmosphäre kann einen im Vergleich zur Luft verringerten Sauerstoffgehalt aufweisen.

Der Gasdruck im Inneren der Hülle kann vorteilhaft weniger als 0,995 bar, weiter vorteilhaft weniger als 0,99 bar, weiter vorteilhaft weniger als 0,98 bar, weiter vorteilhaft weniger als 0,97 bar, weiter vorteilhaft weniger als 0,96 bar, weiter vorteilhaft weniger als 0,95 bar, weiter vorteilhaft weniger als 0,93 bar, weiter vorteilhaft weniger als 0,9 bar, jeweils bei einer Temperatur von 23°C, sein.

Zum Entfernen wenigstens eines Teils von zwischen den wenigstens zwei Lagen vorhandenem Gas kann wenigstens eine der Lagen in Richtung des Strahlenschutzmaterials gepresst werden. Eine oder beide der Lagen können gegen das Strahlenschutzmaterial oder gegen ein optional zwischen den Lagen ebenfalls vorhandenes Formteil oder Trägermaterial gepresst werden.

Wenigstens eine der Lagen kann durch ein bewegliches Element einer Schweißeinrichtung, die die wenigstens zwei Lagen miteinander verbindet, in Richtung des Strahlenschutzmaterials gepresst werden.

Zum Entfernen wenigstens eines Teils von zwischen den wenigstens zwei Lagen vorhandenem Gas können alternativ oder zusätzlich die wenigstens zwei Lagen und das Strahlenschutzmaterial in eine Bearbeitungskammer mit einem relativ zu einem Umgebungsluftdruck reduzierten Gasdruck eingebracht werden.

Das Strahlenschutzelement, das beispielsweise die Form einer Lamelle aufweisen kann, kann durch Vakuumziehen hergestellt werden.

Bei dem Verfahren kann der Gasdruck in der Bearbeitungskammer beim Verbinden der wenigstens zwei Lagen miteinander weniger als 1 bar, vorteilhaft weniger als 0,995 bar, weiter vorteilhaft weniger als 0,99 bar, weiter vorteilhaft weniger als 0,98 bar, weiter vorteilhaft weniger als 0,97 bar, weiter vorteilhaft weniger als 0,96 bar, weiter vorteilhaft weniger als 0,95 bar, weiter vorteilhaft weniger als 0,93 bar, weiter vorteilhaft weniger als 0,9 bar, jeweils bei der Ausführung des Verbindungsvorgangs der beiden Lagen, sein. In der Bearbeitungskammer kann eine Schutzgasatmosphäre herrschen. Die Schutzgasatmosphäre kann einen im Vergleich zur Luft verringerten Sauerstoffgehalt aufweisen.

Die wenigstens zwei Lagen können entlang einer das Strahlenschutzmaterial ganz oder teilweise umlaufenden Verbindungslinie miteinander verbunden werden.

Bei dem Verfahren können die wenigstens zwei Lagen entlang wenigstens dreier Seiten des Strahlenschutzmaterials miteinander verbunden werden, beispielsweise doch Hochfrequenzschweißen.

Bei dem Verfahren können die wenigstens zwei Lagen entlang aller vier Seiten des Strahlenschutzmaterials miteinander verbunden werden, beispielsweise durch Hochfrequenzschweißen.

Die Verbindung entlang der entsprechenden Seiten kann in einem einzigen Arbeitsschritt erfolgen.

Die das Strahlenschutzmaterial ganz oder teilweise umlaufende Verbindungslinie kann im Wesentlichen luftdicht sein.

Die wenigstens zwei Lagen können entlang einer das Strahlenschutzmaterial nicht umlaufenden weiteren Verbindungslinie miteinander verbunden werden.

Die weitere Verbindungslinie kann im Wesentlichen luftdicht sein.

Das Verfahren kann ein Montieren eines Befestigungselements, beispielsweise eines Ösenrings oder eines Befestigungsstabs, im Inneren der weiteren Verbindungslinie aufweisen.

Die weitere Verbindungslinie kann innerhalb eines Bereichs der Hülle angeordnet sein, der von der Verbindungslinie umgeben wird.

Bei dem Verfahren kann zusätzlich zu dem Strahlenschutzmaterial ein Trägermaterial und/oder ein Formteil zwischen den wenigstens zwei Lagen angeordnet werden.

Die wenigstens zwei Lagen können separate erste und zweite kunststoffhaltige Elemente sein.

Die wenigstens zwei Lagen können verschiedene Bereiche eines einzigen kunststoffhaltigen Elements sein, das so um das Strahlenschutzmaterial umgeschlagen werden kann, dass die wenigstens zwei Lagen die Hauptflächen des Strahlenschutzmaterials überdecken.

Das wenigstens eine kunststoffhaltige Element kann eine Kunststofffolie, insbesondere eine Polyurethanfolie aufweisen.

Das wenigstens eine kunststoffhaltige Element kann ein Textil aufweisen. Das wenigstens eine kunststoffhaltige Element kann ein kunststoffbeschichtetes Gewebe oder Gewirke aufweisen. Das wenigstens eine kunststoffhaltige Element kann ein Gewebe oder Gewirke aus Fasern aufweisen, die mit Kunststoff beschichtet sind.

Das wenigstens eine kunststoffhaltige Element oder wenigstens eine der Lagen kann transluzent sein.

Das Verfahren kann ein Anbringen eines Befestigungselements an den wenigstens zwei Lagen zur Befestigung des Strahlenschutzelements an einem Patiententisch oder an einem Strahlenschutzwagen aufweisen.

Das Befestigungselement kann einen Ösenring oder einen Befestigungsstab aufweisen.

Bei dem Verfahren kann der Gasdruck im Inneren der durch die ersten und zweiten Lagen gebildeten Hülle so reduziert werden, dass der Umgebungsluftdruck im Gebrauch des Strahlenschutzelements die Hülle flächig, insbesondere satt anliegend, gegen das Strahlenschutzmaterial und/oder gegen ein in der Hülle optional vorhandenes Formteil oder Trägermaterial presst.

Ein Strahlenschutzelement nach einem Ausführungsbeispiel weist wenigstens ein Strahlenschutzmaterial und eine Hülle mit einem Innenvolumen auf, in dem das wenigstens eine Strahlenschutzmaterial angeordnet ist. Das Innenvolumen weist einen Gasdruck von weniger als 1 bar (gemessen bei 23°C) auf oder enthält ein Schutzgas.

Der Gasdruck im Innenvolumen kann vorteilhaft weniger als 0,995 bar, weiter vorteilhaft weniger als 0,99 bar, weiter vorteilhaft weniger als 0,98 bar, weiter vorteilhaft weniger als 0,97 bar, weiter vorteilhaft weniger als 0,96 bar, weiter vorteilhaft weniger als 0,95 bar, weiter vorteilhaft weniger als 0,93 bar, weiter vorteilhaft weniger als 0,9 bar, jeweils gemessen bei 23°C, sein.

Ein Strahlenschutzelement nach einem Ausführungsbeispiel weist wenigstens ein Strahlenschutzmaterial und eine Hülle mit einem Innenvolumen auf, in dem das wenigstens eine Strahlenschutzmaterial angeordnet ist. Die Hülle liegt flächig an dem wenigstens einen Strahlschutzmaterial an.

Die Hülle kann flächig und satt an den zwei Hauptflächen des Strahlenschutzmaterials anliegen.

Die Hülle kann eine das Strahlenschutzmaterial ganz oder teilweise umlaufende Verbindungslinie zweier Lagen der Hülle aufweisen.

Die Verbindungslinie kann eine Schweißnaht sein.

Die Verbindungslinie kann durchgängig entlang wenigstens dreier Seiten des Strahlenschutzmaterials verlaufen.

Die Verbindungslinie kann durchgängig entlang aller vier Seiten des Strahlenschutzmaterials verlaufen.

Die das Strahlenschutzmaterial ganz oder teilweise umlaufende Verbindungslinie kann im Wesentlichen luftdicht sein.

Die Hülle kann eine das Strahlenschutzmaterial nicht umlaufende weitere Verbindungslinie aufweisen.

Die weitere Verbindungslinie kann im Wesentlichen luftdicht sein.

Die Hülle kann ein Befestigungselement, beispielsweise einen Ösenring oder einen Befestigungsstab, im Inneren der weiteren Verbindungslinie aufweisen.

Die weitere Verbindungslinie kann innerhalb eines Bereichs der Hülle angeordnet sein, der von der Verbindungslinie umgeben ist.

Das Strahlenschutzelement kann zusätzlich zu dem Strahlenschutzmaterial ein Trägermaterial und/oder ein Formteil im Innenvolumen der Hülle aufweisen.

Die Hülle kann eine Kunststofffolie, insbesondere eine Polyurethanfolie aufweisen.

Die Hülle kann ein Textil aufweisen oder aus einem Textil bestehen. Beispielsweise kann die Hülle ein kunststoffbeschichtetes Gewebe oder Gewirke aufweisen oder daraus bestehen. Die Hülle kann eine Gewebe oder Gewirke aus Fasern, die mit Kunststoff beschichtet sind, aufweisen oder daraus bestehen.

Die Hülle kann transluzent sein.

Das Strahlenschutzelement kann ein Befestigungselement aufweisen, das an der Hülle angebracht ist. Das Befestigungselement kann zur Befestigung des Strahlenschutzelements an einem Patiententisch oder an einem Strahlenschutzwagen eingerichtet sein. Das Befestigungselement kann einen Ösenring oder einen Befestigungsstab aufweisen.

Das Strahlenschutzelement kann eine Lamelle sein.

Die Lamelle kann flexibel sein.

Die Lamelle kann starr sein.

Die Lamelle kann beweglich sein.

Die Lamelle kann eine abgewinkelte oder gekrümmte Form aufweisen.

Eine Strahlenschutzeinrichtung nach einem Ausführungsbeispiel weist ein Strahlenschutzelement oder mehrere Strahlenschutzelemente nach einem Ausführungsbeispiel der Erfindung auf.

Die Strahlenschutzeinrichtung kann einen Untertisch-Strahlenschutz aufweisen.

Die Strahlenschutzeinrichtung kann einen Patiententisch mit einer Befestigungseinrichtung aufweisen, an der das Strahlenschutzelement montiert ist oder an der die mehreren Strahlenschutzelemente montiert sind.

Die Befestigungseinrichtung des Patiententischs kann eine Montageschiene sein.

Die Strahlenschutzeinrichtung kann einen Strahlenschutzwagen aufweisen, an dem das Strahlenschutzelement montiert ist oder an dem die mehreren Strahlenschutzelemente montiert sind.

Der Strahlenschutzwagen kann wenigstens ein Strahlenschutzelement nach einem Ausführungsbeispiel als Oberteil aufweisen und/oder als Unterteil aufweisen. Der Strahlenschutzwagen kann wenigstens eine flexible Lamelle als Unterteil aufweisen und wenigstens eine starre oder bewegliche abgewinkelte Lamelle als Oberteil aufweisen.

Bei den Strahlenschutzelement, Herstellungsverfahren und Strahlenschutzeinrichtungen kann das Strahlenschutzmaterial jeweils Bleigummi aufweisen oder Bleigummi sein, ohne darauf beschränkt zu sein. Das Strahlenschutzmaterial kann ein Material mit stark absorbierenden Eigenschaften gegenüber ionisierender Strahlung sein, was durch einen hohen Anteil an stark absorbierenden Elementen (z.B. Blei, Wolfram, Bismut, Antimon) erreicht werden kann, welche in eine Matrixstruktur eingebunden sind.

Verschiedene Wirkungen und Vorteile können mit dem erfindungsgemäßen Herstellungsverfahren, dem erfindungsgemäßen Strahlenschutzelement und der erfindungsgemäßen Strahlenschutzeinrichtung erreicht werden. Durch die Herstellung eines Strahlenschutzelements, bei dem die Hülle flächig satt anliegend gegen das Strahlenschutzmaterial oder Formteile oder Trägermaterialen im Innern der Hülle gepresst wird, wird das Risiko einer Faltenbildung der Hülle reduziert. Dies verringert wiederum das Risiko, dass unerwünschte Falten der Hülle eine korrekte Montage des Strahlenschutzelements erschweren. Oxidationsprozesse können durch Reduzierung der Gasmenge im Innenvolumen der Hülle und/oder durch in der Hülle vorhandenes Schutzgas verringert werden.

Durch das Anpressen der Hülle an das Strahlenschutzmaterial können große Defekte im Strahlenschutzmaterial einfacher erkannt werden, welche beispielsweise durch Alterungsprozesse des Materials oder falsche Handhabung entstehen können.

Eine Sichtprüfung, ob das Strahlenschutzmaterial immer noch korrekt in der Hülle positioniert ist, kann erleichtert werden, wenn die Hülle flächig satt an dem Strahlenschutzmaterial anliegt.

Durch das Anpressen der Hülle an das Strahlenschutzmaterial kann erkannt werden, wo genau sich das in der Hülle angeordnete Strahlenschutzmaterial befindet und somit wo genau der Strahlenschutz gewährleistet wird. Bereiche mit Strahlenschutz zeichnen sich ab.

Die flächig satte Anlage kann insbesondere durch einen Unterdruck im Innenvolumen der Hülle erreicht werden, der bewirkt, dass im Gebrauch des Strahlenschutzelements der Umgebungsluftdruck die Hülle gegen das Strahlenschutzelement oder etwaige im Innenvolumen vorhandene Formteile oder Trägermaterialien presst.

Wenn ein Formteil oder Trägermaterial im Inneren der Hülle angeordnet ist, kann durch den von der Hülle nach innen ausgeübten Druck eine sichere relative Anordnung des Strahlenschutzmaterials relativ zum Formteil oder Trägermaterial sichergestellt werden. Dies kann es je nach Anwendungsfall ermöglichen, das Strahlenschutzmaterial ohne Verwendung von Klebstoff relativ zum Formteil oder Trägermaterial zu fixieren.

### KURZBESCHREIBUNG DER FIGUREN

Nachfolgend wird die vorliegende Erfindung anhand von Ausführungsbeispielen unter Bezugnahme auf die Figuren detailliert erläutert.
Figur 1 ist eine schematische Schnittansicht zur Erläuterung eines Herstellungsverfahrens nach einem Ausführungsbeispiel.
Figur 2 ist eine schematische Schnittansicht zur Erläuterung des Herstellungsverfahrens nach einem Ausführungsbeispiel.
Figur 3 ist eine schematische Draufsicht eines Strahlenschutzelements nach einem Ausführungsbeispiel.
Figur 4 ist eine schematische Schnittansicht des Strahlenschutzelements von Figur 3.
Figur 5 ist eine schematische Draufsicht eines Strahlenschutzelements nach einem weiteren Ausführungsbeispiel.
Figur 6 ist eine schematische Schnittansicht zur Erläuterung des Herstellungsverfahrens nach einem Ausführungsbeispiel.
Figur 7 ist eine schematische Schnittansicht des Strahlenschutzelements, das mit dem unter Bezugnahme auf Figur 6 erläuterten Verfahren herstellbar ist.
Figur 8 ist eine schematische Schnittansicht zur Erläuterung des Herstellungsverfahrens nach einem Ausführungsbeispiel.
Figur 9 ist eine schematische Schnittansicht zur Erläuterung des Herstellungsverfahrens nach einem Ausführungsbeispiel.
Figur 10 ist eine schematische Schnittansicht zur Erläuterung des Herstellungsverfahrens nach einem Ausführungsbeispiel.
Figur 11 ist eine schematische Darstellung einer Strahlenschutzeinrichtung mit einem Strahlenschutzelement nach einem Ausführungsbeispiel.
Figur 12 ist eine schematische Darstellung einer Strahlenschutzeinrichtung mit einem Strahlenschutzelement nach einem Ausführungsbeispiel.

### BESCHREIBUNG BEVORZUGTER AUSFÜHRUNGSBEISPIELE

Nachfolgend werden vorteilhafte oder bevorzugte Ausführungsbeispiele unter Bezugnahme auf die Figuren detailliert beschrieben. Die Merkmale der verschiedenen Ausführungsbeispiele können miteinander kombiniert werden, sofern dies in der nachfolgenden Beschreibung nicht ausdrücklich ausgeschlossen wird. Korrespondierende oder identische Bezugszeichen bezeichnen korrespondierende oder identische Elemente.

Die Herstellungsverfahren und die damit hergestellten Strahlenschutzelemente können bei Strahlenschutzeinrichtungen zur Verwendung beispielsweise in der interventionellen Radiologie eingesetzt werden, mit denen bei Diagnostik und Therapie sowie in Operationsräumen beteiligtes Personal gegen auftretende Strahlung, insbesondere Röntgenstrahlung, geschützt wird.

Die Herstellungsverfahren und die damit hergestellten Strahlenschutzelemente können insbesondere für Lamellen eingesetzt werden, die als Oberteile oder Unterteile von Strahlenschutzeinrichtungen zum Einsatz kommen.

Unterteile derartiger Strahlenschutzeinrichtungen können aus Behängen bestehen, die aus einzelnen breitflächigen oder mehreren, überlappend zueinander angeordneter schmalen Lamellen gebildet werden. Diese sorgen für den Strahlenschutz von einem Tischniveau ab in Richtung Fußboden.

Oberteile können beispielsweise als abgewinkelt oder gekrümmt ausgeformte Lamellen ausgestaltet sein, welche einen Strahlenschutz oberhalb des Tischniveaus unterstützen. Auch das Oberteil kann durch eine einzelne, breite und durchgehende Lamelle gebildet sein, oder das Oberteil kann sich aus mehreren schmalen, zueinander überlappenden Lamellen zusammensetzen.

Nach Ausführungsbeispielen der Erfindung kann ein Strahlenschutzmaterial, beispielsweise ein Bleigummi-Zuschnitt, zwischen zwei Lagen eines kunststoffhaltigen Materials, beispielsweise Kunststoff-Zuschnitte, gelegt werden. Das Strahlenschutzmaterial kann absorbierende chemische Elemente, beispielsweise Blei, Wolfram, Bismut und/oder Antimon, in einer Matrix aufweisen.

Die wenigstens zwei Lagen können zu einer Hülle verbunden werden. Die zwei Lagen können miteinander so verbunden werden, dass die durch Verbinden der zwei Lagen des kunststoffhaltigen Materials erzeugte Hülle flächig eng an dem Strahlenschutzmaterial und/oder optional im Inneren der Hülle zusätzlich vorhandenen Materialien anliegt. Alternativ oder zusätzlich kann im Inneren der Hülle ein Schutzgas vorhanden sein, so dass im Inneren der Hülle ein Sauerstoffgehalt im Vergleich zur Umgebungsluft verringert ist.

Dazu kann die Hülle beispielsweise so gebildet werden, dass bei der Herstellung des Strahlenschutzelements vor Verbinden der wenigstens zwei Lagen miteinander ein Bereich zwischen den wenigstens zwei Lagen teilweise von Gas evakuiert wird und/oder die wenigstens zwei Lagen in einer Schutzgasatmosphäre miteinander verbunden werden. Dies kann in einer besonderen Bearbeitungsatmosphäre, beispielsweise einer Bearbeitungskammer mit Teilvakuum in ihrem Inneren oder in einer Schutzgasatmosphäre, erreicht werden. Alternativ oder zusätzlich kann wenigstens eine der Lagen durch ein bewegliches Maschinenteil flächig gegen das Strahlenschutzmaterial oder ein Formteil gedrückt werden, während die zwei Lagen miteinander verbunden werden.

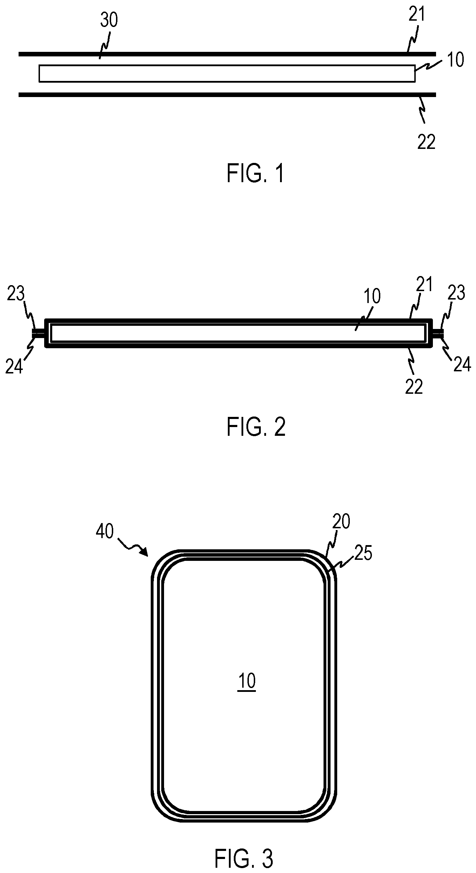

Figur 1 und Figur 2 zeigen schematische Schnittansichten zur Erläuterung des Herstellungsverfahrens.

Wenigstens ein Strahlenschutzmaterial 10 kann zwischen zwei Lagen 21, 22 eines kunststoffhaltigen Materials angeordnet werden. Die zwei Lagen 21, 22 können separate Flächenelemente, beispielsweise Kunststoff-Zuschnitte, sein.

Das Strahlenschutzmaterial 10 kann so gewählt sein, dass es Röntgenstrahlung absorbiert. Beispielsweise kann das Strahlenschutzmaterial 10 aus Bleigummi bestehen oder Bleigummi aufweisen. Das Strahlenschutzmaterial 10 kann eine Matrixstruktur mit darin enthaltenen stark absorbierenden Elementen aufweisen. Die stark absorbierenden Elemente können beispielsweise Blei, Wolfram, Bismut und/oder Antimon aufweisen. Das Strahlenschutzmaterial 10 kann eine Lage oder mehrere Lagen eines flächigen Strahlenschutzmaterials aufweisen.

Die zwei Lagen 21, 22 der Hülle können im Wesentlichen luftdicht sein.

Die zwei Lagen 21, 22 der Hülle können jeweils aus einer Kunststofffolie, beispielsweise aus einer Polyurethanfolie bestehen. Die Kunststofffolie kann eine Dichte größer als 1 g/cm³, vorteilhaft größer als 1,1 g/cm³ aufweisen. Die Kunststofffolie kann eine Dichte kleiner als 1,5 g/cm³, vorteilhaft kleiner als 1,3 g/cm³ aufweisen. Die Kunststofffolie kann eine Dicke kleiner als 3000 µm, vorteilhaft maximal 1000 µm aufweisen. Die Kunststofffolie kann eine Dicke größer als 25 µm, vorteilhaft größer als 100 µm aufweisen.

Die zwei Lagen 21, 22 können alternativ aus einem Gewebe oder Gewirke bestehen, das kunststoffbeschichtet ist. Das Gewebe oder Gewerke kann aus kunststoffbeschichteten Fasern bestehen. Die Lagen 21, 22 können eine Kunststofffolie aufweisen, die mit Fasern gefertigt ist.

Eine oder beide der Lagen 21, 22 kann bzw. können alternativ oder zusätzlich transluzent sein. Dadurch wird eine Überprüfung der richtigen Positionierung des Strahlenschutzmaterials in dem Strahlenschutzelement erleichtert. Beispielsweise kann die Erkennung von größeren Schäden am Strahlenschutzmaterial 10 erleichtert werden, die beispielsweise durch Alterungsprozesse oder unsachgemäße Handhabung hervorgerufen werden können.

Bei der Herstellung des Strahlenschutzelements können die beiden Lagen 21, 22 eng anliegend gegen die Hauptflächen des Strahlenschutzmaterials 10 gedrückt werden, wobei eine im Bereich 30 zwischen den beiden Lagen 21, 22 vorhandene Gasmenge reduziert wird. Durch Andrücken der Lagen 21, 22 und/oder durch Erzeugen eines Teilvakuums im Bereich 30 zwischen den Lagen 21, 22 kann das Risiko einer unerwünschten Faltenbildung in der Hülle 20 des Strahlenschutzelements 40 reduziert werden. In diesem Bearbeitungszustand, in dem die Gasmenge im Bereich 30 reduziert wurde, können die beiden Lagen 21, 22 miteinander verbunden werden.

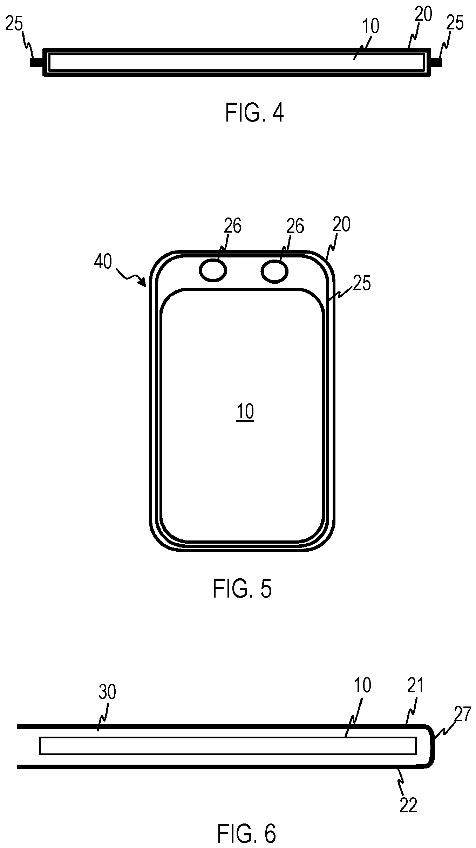

Zum Verbinden der beiden Lagen 21, 22 können die beiden Lagen 21, 22 entlang überlappender Randbereiche 23,24 miteinander verbunden werden, beispielsweise durch Hochfrequenzschweißen. Eine dadurch gebildete Verbindungslinie 25, die am besten in der Draufsicht von Figur 3 und der Schnittansicht des hergestellten Strahlenschutzelements 40 von Figur 4 erkennbar ist, kann das Strahlenschutzmaterial 10 vollständig umlaufen. Es kann eine einzige, das Strahlenschutzmaterial 10 an allen vier Seiten umlaufende Verbindungslinie 25, beispielsweise eine Schweißnaht, gebildet werden.

Die Verbindungslinie 25 kann im Wesentlichen luftdicht sein.

Wie in Figur 5 dargestellt, können die Lagen 21, 22 zusätzlich entlang einer oder mehrerer weiterer Verbindungslinien 26 miteinander verbunden werden. Beispielsweise können Verbindungslinien 26 innerhalb des von der äußeren Verbindungslinie 25 umschlossenen Bereichs gebildet werden, um Befestigungselemente wie Ösenringe oder Befestigungsstäbe innerhalb der weiteren Verbindungslinien 26 anzubringen. Wenn die weiteren Verbindungslinien 26 innerhalb des von der äußeren Verbindungslinie 25 umschlossenen Bereichs gebildet werden, sind weiteren Verbindungslinien 26 vorteilhaft im Wesentlichen luftdicht ausgeführt. Dadurch kann sichergestellt werden, dass im gebildeten Strahlenschutzelement 40 die Hülle 20 auch bei längerem Gebrauch im Wesentlichen faltenfrei bleibt und/oder Schutzgas in der Hülle erhalten bleibt.

Während unter Bezugnahme auf Figuren 1-5 Ausführungsbeispiele beschrieben wurden, bei denen die zwei Lagen 21, 22 durch separate Elemente gebildet werden, die miteinander entlang einer geschlossenen Verbindungslinie 25 verbunden werden, sind auch andere Ausgestaltungen möglich.

Figur 6 ist eine schematische Schnittansicht zur Erläuterung eines weiteren Ausführungsbeispiels. Die zwei Lagen 21, 22 werden durch unterschiedliche Bereiche eines einzigen flächigen Elements gebildet, das beispielsweise entlang eines Falzes 27 so umgeschlagen sein kann, dass die zwei Lagen 21, 22 an entgegengesetzten Hauptflächen des Strahlenschutzmaterials 10 angeordnet sind.

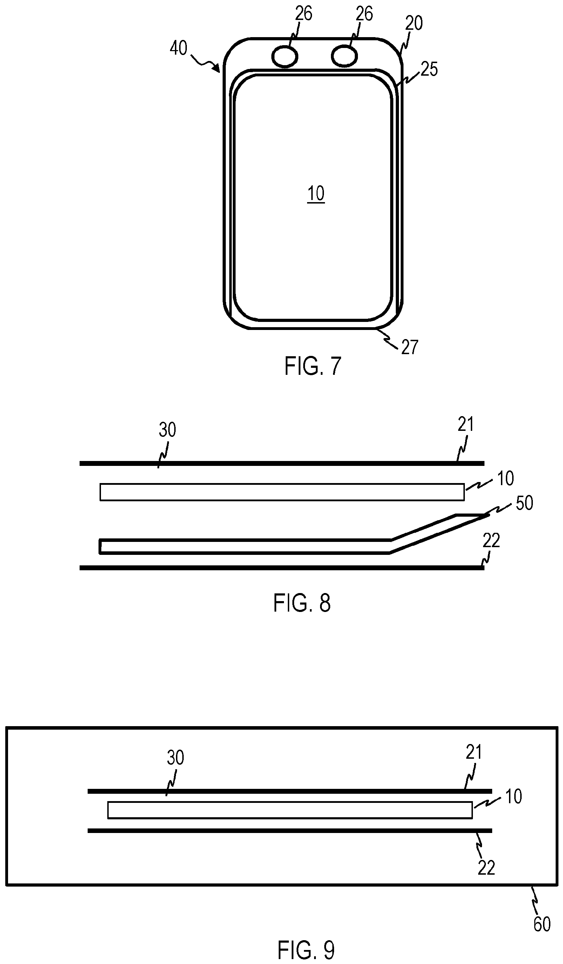

Bei einer derartigen Ausgestaltung ist es nicht zwingend erforderlich, die zwei Lagen 21, 22 entlang einer geschlossenen Linie miteinander zu verbinden. Wie in Figur 7 schematisch dargestellt ist, können die beiden Lagen 21, 22 beispielsweise entlang einer das Strahlenschutzmaterial 10 nur an drei Seiten umgebenden Verbindungslinie 25 miteinander verbunden werden, insbesondere in im Wesentlichen luftdichter Weise. Die Verbindungslinie 25 erstreckt sich bis zu dem Falz 27, an dem das kunststoffhaltigen Material umgeschlagen ist.

Wie ebenfalls schematisch in Figur 7 dargestellt ist, müssen etwaige weitere Verbindungslinien 26, die zur Anbringung von Befestigungselementen, wie Ösenringen oder Befestigungsstäben dienen, nicht zwingend innerhalb des durch die Verbindungslinie 25 definierten Bereichs angeordnet sein. Beispielsweise können die weiteren Verbindungslinien 26 auch außerhalb der durch die Verbindungslinie 25 definierten Regionen positioniert sein. Die weiteren Verbindungslinien 26 müssen nicht zwingend luftdicht sein.

Bei jedem der Ausführungsbeispiele können zusätzlich zu dem Strahlenschutzmaterial 10 weitere Elemente im Inneren der Hülle 20 angeordnet sein, die durch die wenigstens zwei Lagen 21, 22 gebildet wird. Derartige zusätzliche Elemente können der Definition einer dreidimensionalen Form oder anderweitig der mechanischen Stabilisierung dienen.

Figur 8 zeigt beispielhaft eine Ausgestaltung, bei der ein Formteil 50 im Bereich 30 zwischen den zwei Lagen 21, 22 angeordnet wird. Bei der Herstellung des Strahlenschutzelements 40 wird das Strahlenschutzmaterial 10 von der Hülle 20 gegen das Formteil 50 gepresst.

Aufgrund der Erzeugung eines Unterdrucks im Inneren der durch die zwei Lagen 21, 22 gebildeten Hülle 20 ist es möglich, das Strahlenschutzmaterial 10 an dem Formteil 50 zu sichern, ohne dass das Strahlenschutzmaterial 10 dafür zwingend durch Klebstoff an dem Formteil 50 befestigt werden muss. Bei weiteren Ausführungsbeispielen kann optional Klebstoff verwendet werden, um das Strahlenschutzmaterial 10 an dem Formteil 50 anzubringen.

Das Formteil 50 kann aus einem Kunststoff bestehen. Alternativ oder zusätzlich können zusätzlich zu dem Strahlenschutzmaterial 10 andere Trägermaterialien in der Hülle 20 des Strahlenschutzelements 40 angeordnet sein.

Die Herstellung eines Strahlenschutzelements 40 mit reduziertem Gasdruck im Inneren kann auf verschiedene Weise erfolgen.

Wie in Figur 9 dargestellt, können die zwei Lagen 21, 22 in einer Atmosphäre mit reduziertem Luftdruck oder in einer Schutzgasatmosphäre miteinander verbunden werden. Die zwei Lagen 21, 22, das Strahlenschutzmaterial 10 und etwaige vorhandene Formteil 50 oder andere Trägermaterialien können zum Verbinden der beiden Lagen 21, 22 miteinander in eine Bearbeitungskammer 60 eingebracht werden. Die Bearbeitungskammer 60 kann teilweise evakuiert werden, bevor die zwei Lagen 21, 22 miteinander verbunden werden. Alternativ oder zusätzlich kann in der Bearbeitungskammer 60 eine Schutzgasatmosphäre erzeugt werden, bevor die zwei Lagen 21, 22 miteinander verbunden werden.

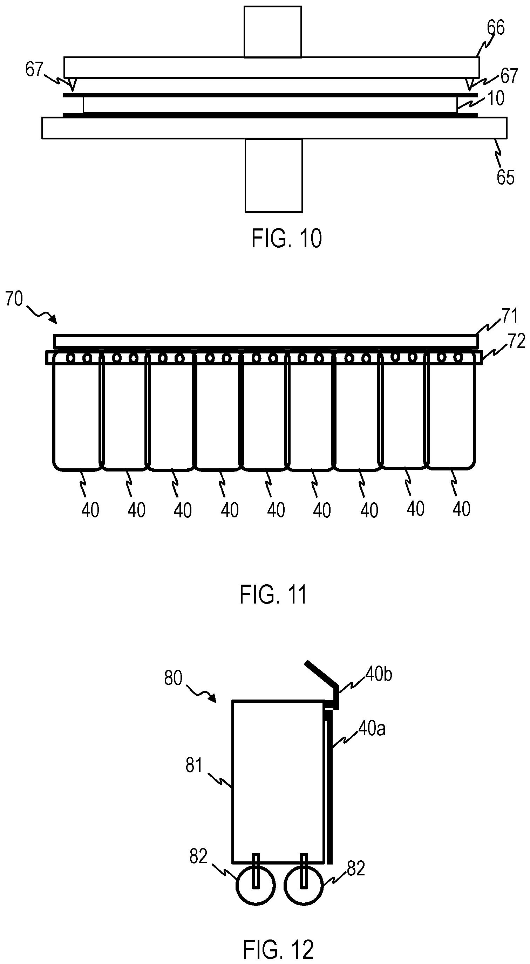

Wie in Figur 10 dargestellt, kann eine Gasmenge im Bereich zwischen den zwei Lagen 21, 22 dadurch verringert werden, dass eine Maschine eine oder beide der Lagen 21, 22 flächig gegen das Strahlenschutzmaterial 10 und/oder optional vorhandene Formteile 50 oder ein optional vorhandenes Trägermaterial presst. Dies kann in einem Bearbeitungswerkzeug für die zwei Lagen 21, 22 erfolgen. Beispielsweise kann eine Einrichtung zum Verschweißen der zwei Lagen 21, 22 eine ortsfeste oder bewegliche Anlagefläche 65 und mindestens ein gegenüber der Anlagefläche 65 bewegliches Maschinenbauteil 66 aufweisen. Sonotroden 67 können zum Hochfrequenzschweißen der Lagen 21, 22 vorgesehen sein. Durch Bewegung der Maschinenteile 65, 66 aufeinander zu können die Lagen 21, 22 gegen das Strahlenschutzmaterial 10 oder andere zwischen die zwei Lagen 21, 22 gelegte Elemente gedrückt werden. Die Gasmenge im Bereich 30 zwischen den zwei Lagen 21, 22 kann dadurch reduziert werden.

Strahlenschutzelemente 40 nach Ausführungsbeispielen können in verschiedenen Strahlenschutzeinrichtungen für den Schutz unterhalb und oberhalb des Patiententisches Verwendung finden.

Figur 11 ist eine schematische Darstellung eines Patiententisches 70 mit einer Patientenliege 71 und einer Montageschiene 72. Ein Strahlenschutzelement 40 oder mehrere Strahlenschutzelemente 40 nach einem Ausführungsbeispiel können an der Montageschiene 72 befestigt sein. Die Strahlenschutzelemente 40 können als Untertisch-Strahlenschutz wirken. Die Strahlenschutzelemente 40 können jeweils als biegsame oder beweglich, insbesondere schwenkbar, angeordnete Lamellen ausgebildet sein. Der Untertisch-Strahlenschutz kann durch eine einzige Lamelle oder mehrere überlappend angeordnete Lamellen gebildet werden.

Figur 12 ist eine schematische Darstellung eines Strahlenschutzwagens 80. Der Strahlenschutzwagen 80 weist ein Gestell 81 und Rollen 82 zum Verfahren des Strahlenschutzwagens 80 auf.

Ein oder mehrere erfindungsgemäße Strahlenschutzelemente 40a können als Unterteile des Strahlenschutzes am Strahlenschutzwagen 80 montiert sein. Die Strahlenschutzelemente 40a können jeweils flexible oder starre Lamellen sein. Die Lamellen können starr oder beweglich montiert sein.

Alternativ oder zusätzlich können ein oder mehrere erfindungsgemäße Strahlenschutzelemente 40b als Oberteile am Strahlenschutzwagen 80 montiert sein. Die Strahlenschutzelemente 40b können ein Formteil im Inneren der Hülle aufweisen, um eine abgewinkelte oder gekrümmte Geometrie zu realisieren.

Verschiedene Wirkungen werden durch die erfindungsgemäßen Strahlenschutzelemente und Verfahren erreicht. Beispielsweise legt sich die Hülle eng an das Strahlenschutzmaterial an. Oxidationsprozesse können aufgrund des geringeren Lufteinschlusses in der Hülle und/oder aufgrund von in der Hülle eingeschlossenem Schutzgas verlangsamt werden. Ein Ausbeulen der Hülle und eine Faltenbildung der Hülle kann reduziert oder vollständig unterbunden werden.

Durch die enge Anlage der Hülle am Strahlenschutzmaterial und optional vorhandenen Trägermaterialien kann eine mechanische Unterstützung des Strahlenschutzmaterials erreicht werden.

Aufgrund des Anpressdrucks der Hülle gegen das Strahlenschutzmaterial und in der Hülle vorhandene Formteile oder andere Trägermaterialien kann optional auf ein Verkleben des Strahlenschutzmaterials mit dem Trägermaterial verzichtet werden.

Erfindungsgemäße Strahlenschutzelemente und die entsprechenden Herstellungsverfahren können für Strahlenschutzelemente verwendet werden, die an einem Patiententisch oder Strahlenschutzwagen montierbar sind. Die erfindungsgemäßen Verfahren und Strahlenschutzelemente sind nicht auf diese spezifische Anwendung beschränkt, sondern können beispielsweise auch bei Schutzkleidung zum Einsatz kommen.

Erfindungsgemäße Strahlenschutzelemente und die entsprechenden Herstellungsverfahren können für einen Schutz gegen Röntgenstrahlung eingesetzt werden, insbesondere bei Verfahren der interventionellen Radiologie, ohne hierauf beschränkt zu sein.

## Patentansprüche

1. Strahlenschutzelement (40; 40a, 40b), aufweisend:
wenigstens ein Strahlenschutzmaterial (10) und
eine Hülle (20) mit einem Innenvolumen, in dem das wenigstens eine Strahlenschutzmaterial (10) angeordnet ist, **gekennzeichnet dadurch, dass**
das Innenvolumen einen Gasdruck von weniger als 1 bar bei 23°C aufweist oder
das Innenvolumen ein Schutzgas aufweist.

2. Strahlenschutzelement nach Anspruch 1,
wobei die Hülle (20) flächig an dem wenigstens einen Strahlschutzmaterial (10) anliegt.

3. Strahlenschutzelement nach Anspruch 1 oder Anspruch 2, wobei die Hülle (20) eine das Strahlenschutzmaterial (10) ganz oder teilweise umlaufende Verbindungslinie (25) zweier Lagen (21, 22) der Hülle (20) aufweist.

4. Strahlenschutzelement nach einem der vorhergehenden Ansprüche,
wobei die Hülle (20) eine das Strahlenschutzmaterial (10) nicht umlaufende weitere Verbindungslinie (26) zweier Lagen (21, 22) der Hülle (20) aufweist, wobei die weitere Verbindungslinie (26) eine Öffnung in der Hülle (20) umläuft.

5. Strahlenschutzeinrichtung (70; 80), aufweisend:
ein Strahlenschutzelement (40; 40a, 40b) oder mehrere Strahlenschutzelemente (40; 40a, 40b) nach einem der vorhergehenden Ansprüche.

6. Strahlenschutzeinrichtung (70; 80) nach Anspruch 5, wobei die Strahlenschutzeinrichtung einen Patiententisch (70) mit einer Befestigungseinrichtung (72) aufweist, an der für einen Schutz unterhalb oder oberhalb des Patiententisches (70) das Strahlenschutzelement (40; 40a, 40b) montiert ist oder an der für einen Schutz unterhalb oder oberhalb des Patiententisches (70) die mehreren Strahlenschutzelemente (40; 40a, 40b) montiert sind, oder wobei die Strahlenschutzeinrichtung einen Strahlenschutzwagen (80) aufweist, an dem das Strahlenschutzelement (40a, 40b) montiert ist oder an dem die mehreren Strahlenschutzelemente (40a, 40b) montiert sind.

7. Verfahren zum Herstellen eines Strahlenschutzelements (40; 40a, 40b) nach einem der Ansprüche 1 bis 4, mit den Schritten:
Anordnen wenigstens eines Strahlenschutzmaterials (10) zwischen wenigstens zwei Lagen (21, 22) wenigstens eines kunststoffhaltigen Elements,
Entfernen wenigstens eines Teils von zwischen den wenigstens zwei Lagen (21, 22) vorhandenem Gas oder Positionieren der wenigstens zwei Lagen (21, 22) mit dem dazwischen angeordnete Strahlenschutzmaterial (10) in einer Schutzgasatmosphäre, und
Verbinden der wenigstens zwei Lagen (21, 22) miteinander.

8. Verfahren nach Anspruch 7, wobei in einem Bereich (30) zwischen den wenigstens zwei Lagen (21, 22) ein Gasdruck von einem Ausgangsdruck auf einen Bearbeitungsgasdruck reduziert ist, während die wenigstens zwei Lagen (21, 22) miteinander verbunden werden.

9. Verfahren nach Anspruch 8, wobei der Bearbeitungsgasdruck so gewählt ist, dass im Inneren einer durch Verbinden der wenigstens zwei Lagen (21, 22) gebildeten Hülle (20) des Strahlenschutzmaterials (10) bei 23°C ein Gasdruck von weniger als 1 bar herrscht.

10. Verfahren nach einem der Ansprüche 7 bis 9, wobei zum Entfernen wenigstens eines Teils von zwischen den wenigstens zwei Lagen (21, 22) vorhandenem Gas wenigstens eine der Lagen in Richtung des Strahlenschutzmaterials (10) gepresst wird,
wobei optional wenigstens eine der Lagen (21, 22) durch ein bewegliches Element (66) einer Schweißeinrichtung zum Verbinden der wenigstens zwei Lagen (21, 22) in Richtung des Strahlenschutzmaterials (10) gepresst wird.

11. Verfahren nach einem der Ansprüche 7 bis 10, wobei die wenigstens zwei Lagen (21, 22) und das Strahlenschutzmaterial (10) in eine Bearbeitungskammer (60) mit einem relativ zu einem Umgebungsgasdruck reduzierten Gasdruck oder mit einer Schutzgasatmosphäre eingebracht werden.

12. Verfahren nach einem der Ansprüche 7 bis 11, wobei die wenigstens zwei Lagen (21, 22) entlang einer das Strahlenschutzmaterial (10) ganz oder teilweise umlaufenden Verbindungslinie (25) miteinander verbunden werden,
wobei optional die wenigstens zwei Lagen (21, 22) entlang einer das Strahlenschutzmaterial (10) nicht umlaufenden weiteren Verbindungslinie (26) miteinander verbunden werden.

13. Verfahren nach einem der Ansprüche 7 bis 12, wobei zusätzlich ein Trägermaterial (50) und/oder ein Formteil (50) zwischen die wenigstens zwei Lagen (21, 22) eingebracht wird, und/oder
wobei das wenigstens eine kunststoffhaltige Element wenigstens ein kunststoffhaltiges Gewebe oder eine Polyurethanfolie aufweist.

14. Verfahren nach einem der vorhergehenden Ansprüche 7 bis 13, wobei das wenigstens eine kunststoffhaltige Element oder wenigstens eine der zwei Lagen (21, 22) transluzent ist.

15. Verfahren nach einem der Ansprüche 7 bis 14, ferner mit dem Schritt: Anbringen eines Befestigungselements an den wenigstens zwei Lagen (21, 22) zur Befestigung des Strahlenschutzelements (40; 40a, 40b) an einem Patiententisch für einen Schutz unterhalb oder oberhalb des Patiententisches oder an einem Strahlenschutzwagen.

## Claims

1. A radiation protection element (40; 40a, 40b), comprising:
at least one radiation protection material (10), and
a sheath (20) with an internal volume in which the at least one radiation protection material (10) is arranged, **characterized in that** the internal volume has a gas pressure of less than 1 bar at 23° C or **in that** the internal volume comprises an inert gas.

2. The radiation protection element according to claim 1,
wherein the sheath (20) planarly abuts the at least one radiation protection material (10).

3. The radiation protection element according to claim 1 or claim 2, wherein the sheath (20) comprises a connecting line (25) of two layers (21, 22) of the sheath (20), said connecting line (25) completely or partially extending around the radiation protection material (10).

4. The radiation protection element according to any one of the preceding claims, wherein the sheath (20) comprises an additional connecting line (26) of two layers (21, 22) of the sheath (20), said additional connecting line (26) not extending around the radiation protection material (10), wherein the additional connecting line (26) runs around an opening within the sheath (20).

5. A radiation protection apparatus (70; 80), comprising:
a radiation protection element (40; 40a, 40b) or a plurality of radiation protection elements (40; 40a, 40b) according to any one of the preceding claims.

6. The radiation protection apparatus (70; 80) according to claim 5, wherein the radiation protection apparatus comprises a patient table (70) with a fixing apparatus (72) to which the radiation protection element (40; 40a, 40b) is mounted for protection below or above the patient table (70) or to which the plurality of radiation protection elements (40; 40a, 40b) is mounted for protection below or above the patient table (70), or wherein the radiation protection apparatus comprises a radiation protection cart (80) to which the radiation protection element (40a, 40b) is mounted or to which the plurality of radiation protection elements (40a, 40b) is mounted.

7. A method for producing a radiation protection element (40; 40a, 40b) according to any one of claims 1 to 4, comprising the steps of:
arranging at least one radiation protection material (10) between at least two layers (21, 22) of at least one plastic-containing element,
removing at least part of the gas present between the at least two layers (21, 22) or positioning the at least two layers (21, 22) with the radiation protection material (10) arranged therebetween in an inert gas atmosphere, and
connecting the at least two layers (21, 22) with each other.

8. The method according to claim 7, wherein in a region (30) between the at least two layers (21, 22) a gas pressure is reduced from an initial pressure to a processing gas pressure while the at least two layers (21, 22) are being connected with each other.

9. The method according to claim 8, wherein the processing gas pressure is selected such that inside a sheath (20) of the radiation protection material (10) formed by connecting the at least two layers (21, 22) a gas pressure is less than 1 bar at 23° C.

10. The method according to any one of claims 7 to 9, wherein at least one of the layers is pressed toward the radiation protection material (10) in order to remove at least part of the gas present between the at least two layers (21, 22), wherein optionally at least one of the layers (21, 22) is pressed toward the radiation protection material (10) by means of a movable element (66) of a welding apparatus in order to connect the at least two layers (21, 22).

11. The method according to any one of claims 7 to 10, wherein the at least two layers (21, 22) and the radiation protection material (10) are placed in a processing chamber (60) with a gas pressure reduced relative to an ambient gas pressure or with an inert gas atmosphere.

12. The method according to any one of claims 7 to 11, wherein the at least two layers (21, 22) are connected with each other along a connecting line (25) completely or partially extending around the radiation protection material (10),wherein optionally the at least two layers (21, 22) are connected with each other along an additional connecting line (26) not extending around the radiation protection material (10).

13. The method according to any one of claims 7 to 12, wherein a carrier material (50) and/or a shaped part (50) is additionally incorporated between the at least two layers (21, 22), and/or wherein the at least one plastic-containing element comprises at least one plastic-containing woven fabric or a polyurethane film.

14. The method according to any one of claims 7 to 13, wherein the at least one plastic-containing element or at least one of the two layers (21, 22) is translucent.

15. The method according to any one of claims 7 to 14, further comprising the step of attaching a fixing member to the at least two layers (21, 22) for mounting the radiation protection element (40; 40a, 40b) to a patient table for protection below or above the patient table or to a radiation protection cart.

## Revendications

1. Elément de radioprotection (40 ; 40a, 40b), présentant :
au moins un matériau de radioprotection (10) et
une coque (20) avec un volume intérieur, dans lequel l'au moins un matériau de radioprotection (10) est disposé, **caractérisé en ce que**
le volume intérieur présente une pression de gaz inférieure à 1 bar à 23 °C ou
le volume intérieur présente un gaz de protection.

2. Elément de radioprotection selon la revendication 1,
dans lequel la coque (20) repose à plat sur l'au moins un matériau de radioprotection (10).

3. Elément de radioprotection selon la revendication 1 ou la revendication 2, dans lequel la coque (20) présente une ligne de liaison (25) à deux couches (21, 22) de la coque (20) entourant en totalité ou en partie le matériau de radioprotection (10).

4. Elément de radioprotection selon l'une quelconque des revendications précédentes, dans lequel la coque (20) présente une autre ligne de liaison (26) à deux couches (21, 22) de la coque (20) n'entourant pas le matériau de radioprotection (10), dans lequel l'autre ligne de liaison (26) entoure une ouverture dans la coque (20).

5. Dispositif de radioprotection (70 ; 80) présentant :
un élément de radioprotection (40 ; 40a, 40b) ou plusieurs éléments de radioprotection (40 ; 40a, 40b) selon l'une quelconque des revendications précédentes.

6. Dispositif de radioprotection (70 ; 80) selon la revendication 5, dans lequel le dispositif de radioprotection présente une table pour patient (70) avec un dispositif de fixation (72), sur lequel l'élément de radioprotection (40 ; 40a, 40b) est monté en dessous ou au-dessus de la table pour patient (70) pour une protection ou sur lequel les plusieurs éléments de radioprotection (40 ; 40a, 40b) sont montés en dessous ou au-dessus de la table pour patient (70) pour une protection, ou dans lequel le dispositif de radioprotection présente un chariot de protection (80), sur lequel l'élément de radioprotection (40a, 40b) est monté ou sur lequel les plusieurs éléments de radioprotection (40a, 40b) sont montés.

7. Procédé de fabrication d'un élément de radioprotection (40 ; 40a, 40b) selon l'une quelconque des revendications 1 à 4, avec les étapes :
de disposition d'au moins un matériau de radioprotection (10) entre au moins deux couches (21, 22) d'au moins un élément contenant de la matière plastique,
de retrait d'au moins une partie du gaz présent entre les au moins deux couches (21, 22) ou de positionnement des au moins deux couches (21, 22) avec le matériau de radioprotection (10) disposé de manière intercalée sous une atmosphère de gaz de protection, et
de liaison des au moins deux couches (21, 22) l'une à l'autre.

8. Procédé selon la revendication 7, dans lequel une pression de gaz est réduite, dans une zone (30) entre les au moins deux couches (21, 22), d'une pression de départ sur une pression de gaz de traitement, tandis que les au moins deux couches (21, 22) sont reliées l'une à l'autre.

9. Procédé selon la revendication 8, dans lequel la pression de gaz de traitement est choisie de telle sorte qu'une pression de gaz inférieure à 1 bar règne à l'intérieur d'une coque (20), formée par liaison des au moins deux couches (21, 22), du matériau de radioprotection (10) à 23 °C.

10. Procédé selon l'une quelconque des revendications 7 à 9, dans lequel au moins une des couches est pressée en direction du matériau de radioprotection (10) pour retirer au moins une partie du gaz présent entre les au moins deux couches (21, 22),
dans lequel en option au moins une des couches (21, 22) est pressée par un élément mobile (66) d'un dispositif de soudage pour relier les au moins deux couches (21, 22) en direction du matériau de radioprotection (10).

11. Procédé selon l'une quelconque des revendications 7 à 10, dans lequel les au moins deux couches (21, 22) et le matériau de radioprotection (10) sont introduits dans une chambre de traitement (60) avec une pression de gaz réduite par rapport à une pression de gaz environnante ou avec une atmosphère de gaz de protection.

12. Procédé selon l'une quelconque des revendications 7 à 11, dans lequel les au moins deux couches (21, 22) sont reliées l'une à l'autre le long d'une ligne de liaison (25) entourant en totalité ou en partie le matériau de radioprotection (10),
dans lequel en option les au moins deux couches (21, 22) sont reliées l'une à l'autre le long d'une autre ligne de liaison (26) n'entourant pas le matériau de radioprotection (10).

13. Procédé selon l'une quelconque des revendications 7 à 12, dans lequel en supplément un matériau de support (50) et/ou une partie moulée (50) sont introduits entre les au moins deux couches (21, 22), et/ou
dans lequel l'au moins un élément contenant de la matière plastique présente au moins un tissu contenant de la matière plastique ou un film de polyuréthane.

14. Procédé selon l'une quelconque des revendications précédentes 7 à 13, dans lequel l'au moins un élément contenant de la matière plastique ou au moins une des deux couches (21, 22) est translucide.

15. Procédé selon l'une quelconque des revendications 7 à 14, avec en outre l'étape :
d'installation d'un élément de fixation sur les au moins deux couches (21, 22) pour la fixation de l'élément de radioprotection (40 ; 40a, 40b) sur une table pour patient pour une protection en dessous ou au-dessus de la table pour patient ou sur un chariot de radioprotection.
